(19) Europäisches Patentamt
European Patent Office
Office européen des brevets

(11) Publication number: 0 228 484
A1

# (12) EUROPEAN PATENT APPLICATION

(21) Application number: **85308699.9**

(22) Date of filing: **29.11.85**

(51) Int. Cl.[4]: **C07C 29/38**, C07C 31/20

(43) Date of publication of application:
**15.07.87 Bulletin 87/29**

(84) Designated Contracting States:
**BE DE FR GB IT**

(71) Applicant: **TEXACO DEVELOPMENT CORPORATION**
**2000 Westchester Avenue**
**White Plains New York 10650(US)**

(72) Inventor: **Knifton, John Frederick**
**10900 Catskill Trail**
**Austin Texas 78761(US)**
Inventor: **Duranleau, Roger George**
**Route 5 Box 87**
**Georgetown Texas 78626(US)**
Inventor: **Lin, Jiang-Jen**
**2617 Oak Meadow Drive**
**Round Rock Texas 78664(US)**
Inventor: **Grice, Neal John**
**24003 Akron Cove**
**Austin Texas 78723(US)**

(74) Representative: **Burnside, Michael et al**
**Michael Burnside & Partners 2 Serjeants' Inn**
**Fleet Street**
**London EC4Y 1HL(GB)**

## (54) Production of mixtures containing ethylene glycol from synthesis gas.

(57) A process for the production of mixtures containing ethylene glycol by reacting carbon monoxide, hydrogen and formaldehyde or 1,3-dioxolane at a temperature of at least 50°C and a pressure of at least 3.5 MPa in the presence of a cobalt catalyst, characterised in that the catalyst is a homogeneous liquid catalyst comprising cobalt, a halogen-free silicon compound having at least one carbon-silican bond, and a solvent comprising an aromatic hydrocarbon or halohydrocarbon, a halo aromatic ether, a cyclic ether, or an excess of 1,3-dioxolane reactant.

EP 0 228 484 A1

## PRODUCTION OF MIXTURES CONTAINING ETHYLENE GLYCOL FROM SYNTHESIS GAS

This invention relates to a new process for preparing mixtures containing ethylene glycol. More particularly, this invention relates to a novel process for preparing ethylene glycol which comprises contacting syngas (i.e. a mixture of carbon monoxide and hydrogen) with formaldehyde or 1,3-dioxolane in the presence of a catalyst comprising a cobalt compound and certain halogen-free silicon-containing promoters and solvent at a temperature of at least 50°C and a pressure of at least 3.5 MPa.

Ethylene glycol is a chemical which has found wide use in industry. It is used, for example, in the preparation of plasticizers for vinyl polymers and as a component in polyester fibres and antifreeze formulations. In view of its many uses, there is a need to find new and more economical methods for preparing ethylene glycol.

Proposed methods for making ethylene glycol involve the reaction of carbon monoxide with hydrogen in the presence of various proposed catalyst systems at elevated temperatures and pressures. For example, US-A-2 451 333 discloses heating formaldehyde, carbon monoxide and hydrogen with a reduced cobalt oxide hydrogenation catalyst under a pressure in excess of 100 atm. (10.13 MPa) and at a temperature from 80 to 300°C. Actually the examples in this patent used high pressures in the range of 500-800 atmospheres (50.66 to 81.06 MPa).

JP-A-76 128 903 discloses the preparation of ethylene glycol by the reaction of CO, $H_2$ and HCHO with a cobalt catalyst containing a trivalent P, As or Sb compound at a temperature of about 160°C and a pressure of about 180 $Kg/cm^2$.

US-A-4 144 401 uses CO, $H_2$ and formaldehyde as starting materials, but they are reacted in the presence of an alcohol solvent and a catalytic amount of rhodium catalyst. Of course use of rhodium in a catalyst makes it expensive for commercial purposes. Methanol is also produced in substantial amounts in this process.

US-A-4 200 765 discloses a process for preparing glycol aldehyde by reacting formaldehyde, hydrogen and carbon monoxide in an aprotic solvent at elevated temperatures and pressures in the presence of a rhodium catalyst, with subsequent hydrogenation of the glycol aldehyde to ethylene glycol.

JP-A-82 118 527 discloses the use of a ruthenium-based catalyst with a trivalent phosphorus compound to convert formaldehyde, CO and $H_2$ into ethylene glycol. The selectivity to ethylene glycol is not specified.

JP-A-82 130 940 employs a rhodium compound and an alkali metal compound. Again selectivity to ethylene glycol is not specified.

In the process of US-A-4 362 820, only carbon monoxide and hydrogen, without formaldehyde, are converted into ethylene glycol using a catalyst comprising a cobalt compound and a large excess of organosilicon compound. In most of the examples, an operating temperature range of 250 to 270°C is employed, coupled with pressures of about 4000 to 8000 psi (27.7 to 55.3 MPa). Weight ratios of ethylene glycol to methanol were typically around 2:1.

JP-A-197909 discloses the use of a solution of formalin carbon monoxide and hydrogen to produce ethylene glycol in the presence of a cobalt catalyst. According to JP-188137, ethylene glycol is produced by reacting CO and hydrogen, optionally with formaldehyde, in the presence of a cobalt carbonyl and phenol and/or alkylphenol.

JP-004782 (1981) discloses a process for producing ethylene glycol from formaldehyde, CO and $H_2$ in the presence of a catalyst containing ruthenium and a trivalent organo-phosphorus compound.

Finally according to JP-57 130 933, acetals are reacted with CO and H in the presence of a cobalt-iodine catalyst system to produce ethylene glycol.

Many of these processes require the use of high pressures (particularly in the absence of an added formaldehyde source), some use expensive rhodium-containing compounds; and in most the selectivities for ethylene glycol are not very high.

The problem solved by the present invention concerns producing ethylene glycol from syngas and formaldehyde by reacting the starting materials in the presence of a catalyst which would be relatively inexpensive, even on a commercial scale, and which could be reacted at low temperatures and pressures, thereby allowing for less expense in construction of reactors, with a selectivity for ethylene glycol, which may be better than found in previous work.

This invention concerns a process for making mixtures containing ethylene glycol by reacting carbon monoxide, hydrogen and formaldehyde with a catalyst comprising a cobtal-containing compound at a temperature of at least 50°C and a pressure of at least 3.5 MPa in the presence of a cobalt catalyst, wherein the catalyst is a homogeneous liquid catalyst comprising cobalt, a halogen-free silicon compound having at least one carbon-silicon bond, and a solvent comprising an aromatic hydrocarbon or halohydrocarbon, a halo aromatic ether, a cyclic ether, or an excess of 1,3-dioxolane reactant. By using this catalyst system one can obtain substantial selectivity in the formation of ethylene glycol, the process can be operated at lower temperatures and pressures and the use of extreme conditions and expensive catalyst compounds required in many of the known processes can be avoided.

The process of the invention as far as the formation of the desired ethylene glycol is concerned may be represented by the following equations:

(1) $CO + 2H_2 + HCHO \rightarrow HOCH_2CH_2OH + H_2O$

(2) 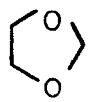 $+ CO + 2H_2 + H_2O \longrightarrow 2HOCH_2CH_2OH$

In order to present the inventive concept of the present invention in the greatest possible detail, the following supplementary disclosure is submitted. The process of the invention is practiced as follows:

As noted, the new catalyst system used in the process of the invention contains a cobalt compound and a silicon-containing promoter. The cobalt compound to be used may be chosen from a wide variety of organic and inorganic compounds, complexes, etc. It is only necessary that the catalyst employed contain the cobalt in any of its ionic states.

The cobalt-containing compound employed may take many different forms. For instance the cobalt may be added to the reaction mixture in an oxide form as in the case of, for example, cobalt(II) oxide, (CoO) or cobalt(II,III) oxide ($Co_3O_4$). Alternatively, it may be added as the salt of a mineral acid, as in the case of cobalt(II) nitrate hydrate ($Co(NO_3)_2 \bullet 6H_2O$) cobalt(II) phosphate, cobalt(II) sulfate, etc. or as the salt of a suitable organic carboxylic acid, for example, cobalt(II) formate, cobalt(II) acetate, cobalt(II) propionate, cobalt naphthenate, or bonded to a carbonyl-containing ligand as in the case of cobalt acetylacetonate, etc. The cobalt may also be added to the reaction zone as a carbonyl or hydridocarbonyl derivative. Here, suitable examples include dicobalt octacarbonyl ($Co_2(CO)_8$), cobalt hydridocarbonyl ($HCo(CO)_4$) and substituted carbonyl species such as the organophosphorus cobalt carbonyls like $HCo(CO)_3(Bu_3P)$.

Preferred cobalt-containing compounds include oxides of cobalt, cobalt salts of minerals acid, cobalt salts of organic carboxylic acids and cobalt carbonyl or hydridocarbonyl derivatives. Among these, particularly preferred are dicobalt octacarbonyl, cobalt(II) oxide, cobalt(II) nitrate, cobalt acetylacetonate and cobalt(II) acetate.

The silicon-containing promoter employed in the practice of this invention is a halogen-free silicon compound having at least one bond between a silicon atom and a carbon atom, but suitable organosilicon compounds may comprise one, two, three or four organo groups bonded to silicon. Each organo group may be an alkyl, aryl or aryalkyl group having 1 to 20 carbon atoms. The silicon-containing promoter may also contain silica-oxygen bonds, and preferred promoters are halogen-free silanes containing at least one silicon-hydrogen bond per molecule.

Typical of suitable organosilicon compounds are trialkylsilanes, such as triethylsilane ($Et_3SiH$), tricyclohexylsilane [$(C_6H_{11})_3SiH$], trimethylsilane, tri-n-hexylsilane and methyl, diethylsilane ($MeEt_2SiH$), as well as dimethylethylsilane and the tripropylsilanes, the dialkylsilanes such as diethylsilane ($Et_2SiH_2$) and dimethylsilane, the tetraalkylsilanes such as tetramethylsilane and tetraethylsilane, the arylsilanes such as triphenylsilane ($Ph_3SiH$), diphenylsilane and hydroxytriphenylsilane, as well as the alkoxysilanes such as triethoxysilane [$(EtO)_3SiH$], phenyltriethoxysilane, tetraethoxysilane and tetramethoxysilane.

Other suitable organosilicon promoters containing at least one silicon-hydride bond, and more than one silicon atom per molecule, include:

$H_3SiCH_2SiH_3$

$H_3SiCH_2CH_2SiH_3$

$CH_3SiH_2CH_2SiH_3$

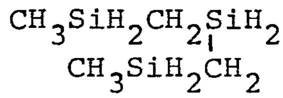

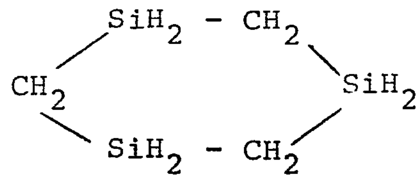

Suitable silanes containing more than one silicon-hydrogen bond per molecule are exemplified by:

$(CH_2=CHCH_2)_2SiH_2$

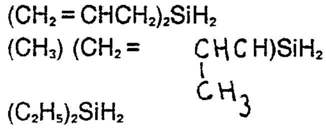

$(C_2H_5)_2SiH_2$

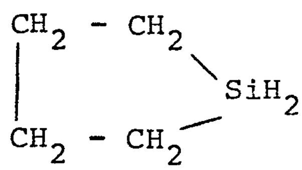

$C_6H_{13}SiH_3$

$CH_3CH=CHCH_2SiH_3$

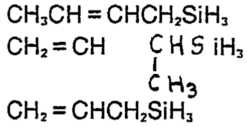

$CH_2=CHCH_2SiH_3$

$C_6H_5CH_2CH_2SiH_3$

$C_6H_5CH(CH_3)SiH_3$

$(C_3H_7)_2SiH_2$

$(CH_3)\ (isoC_4H_9)SiH_2$

$(C_2H_5\ (isoC_4H_9)SiH_2$

$(CH_2=CH)\ (C_2H_5)SiH_2$

$(CH_2=CH)\ (C_4H_9)SiH_2$

Also effective as silicon-containing promoters in the practice of this process are siloxanes, and polyalkylsiloxanes. These may include hexaethyldisiloxane, hexamethylcyclotrisiloxane, octamethylcyclotetrasiloxane, hexamethyldisiloxane, tetramethyldisiloxane ($Me_2HSiOSiHMe_2$), methylhydrocyclosiloxane, as well as alkylsiloxane polymers of the type:

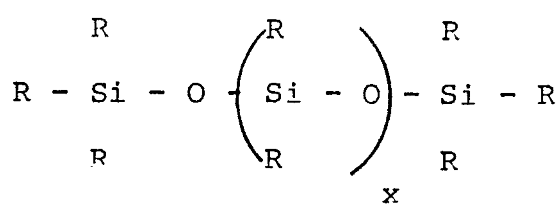

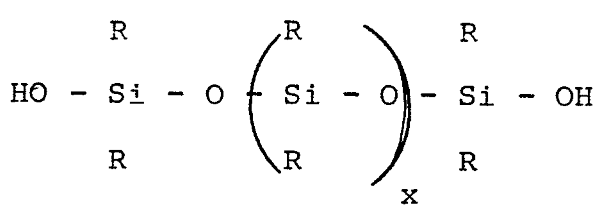

wherein each R, which may be the same or different, is an alkyl group having 1 to 6 carbon atoms.

Equally useful are the higher molecular weight tetraalkylsilanes wherein each alkyl group contains 1 to 20 carbon atoms, and each alkyl group may be the same or different.

Preferred organosilane compounds include triethylsilane, triphenylsilane, trimethylsilane, diphenylsilane, tricyclohexylsilane, tetramethylsilane, tetraethylsilane, hydroxytriphenylsilane, diethylsilane and tripropylsilane.

The homogeneous liquid phase catalyst also comprises a solvent. The solvent may be solid at room temperature but should at least, in part, be a liquid under the conditions of reaction. The solvent is selected from the following materials such that the cobalt catalyst remains in the homogeneous liquid phase throughout the reaction.

The solvent is an aromatic hydrocarbon or halohydrocarbon, a haloaromatic ether, a cyclic ether or, when the reactants include 1,3-dioxolane, an excess of this compound may be used as solvent.

The solvent must be substantially inert under typical co-hydrogenation conditions that yield glycol products and it must be one which has a normal boiling point of at least 65°C at atmospheric pressure. Preferably, the solvent will have a boiling point greater than that of methanol and other oxygen-containing reaction products so that recovery of the glycol products by distillation is facilitated.

Suitable aromatic-type solvents that are satisfactory in the practice of this invention contain 6 to 20 carbon atoms per molecule and at least one aromatic ring moiety per molecule. They are exemplified by, but not limited to, benzene, toluene, p-xylene, o-xylene, m-xylene, mixed xylenes, ethylbenzene, mesitylene, biphenyl, cumene, diethylbenzene, diphenylmethane, dixylylethane, durene, ethyltoluenes, fluorene, naphthalene, n-nonylbenzene, phenyltoluenes, stilbene, tetrahydronaphthalene, tetramethylbenzenes, tetraphenylmethane, and n-propylbenzene.

Preferred aromatic-type solvents include benzene, toluene, m-xylene, o-xylene, p-xylene and tetrahydronaphthalene, as well as mixtures thereof.

Suitable haloaromatic solvents that are satisfactory for this glycol process contain up to 20 carbon atoms and preferably no more than 4 halogen atoms per molecule. They are exemplified by, but not limited to, chlorobenzene, bromobenzene, o-dichlorobenzene, 1,2,4-trichlorobenzene, 1,3,5-tribromobenzene, p-bromotoluene, 2-bromo-m-xylene, α-bromo-p-xylene, 2-chlorobiphenyl, 7-chlorobiphenyl, o-dibromobenzene, 2,2'-dibromobiphenyl, 4,4'-dibromobiphenyl, 2,5-dibromotoluene, 2,4,6-tribromotoluene, 2,3,6-trichlorotoluene, p-xylene dichloride and 1,2,4-trifluorobenzene.

Also effective are haloaromatic ethers. Examples of such solvents include p-bromoanisole, m-chloroanisole and brominated diphenyl oxide. Preferred cyclic ethers are 1,4-dioxane and 1,3-dioxane. Other suitable ether solvents include tetrahydrofuran.

The yield and selectivity of ethylene glycol obtained in the process of the invention depend upon the solvent employed, and upon whether formaldehyde or 1,3-dioxolane is employed as co-reactant.

When formaldehyde (optionally in solid form, such as paraldehyde) is employed as co-reactant, and the solvent is a cyclic ether, typical concentrations of ethylene glycol in the crude liquid product range up to 8.6 wt%, typical yields of ethylene glycol (basis formaldehyde charged) range up to 27 mol%. Total glycol products may comprise up to 18.9 wt% of the crude liquid, and the yields of total glycol products (basis formaldehyde charged) may exceed 50 mol%.

With formaldehyde or paragormaldehyde as co-reactant, and an aromatic hydrocarbon, halohydrocarbon or ether solvent, typical concentrations of ethylene glycol in the crude liquid product phase range up to 24 wt% or more and typical yields of ethylene glycol (basis formaldehyde charged) range up to 30 mol%. Total glycol products may comprise up to 37 wt% or more of the crude liquid. With toluene as solvent, the ethylene glycol concentration in the aqueous product phase is 37 wt%, and the yield of total glycol products is 55 wt%.

With 1,3-dioxolane as co-reactant and an aromatic halohydrocarbon, typical concentrations of ethylene glycol in the crude liquid product range up to 60 wt% of the phase which separates, typical yields of ethylene glycol (basis 1,3-dioxolane charged) range up to 50 mol%. Total glycol products may comprise up to 65 wt% or more of the crude liquid product phase.

When 1,3-dioxolane is used simultaneously as coreactant and as solvent, typical concentrations of ethylene glycol in the crude liquid product range up to 69.1 wt%, typical yields of ethylene glycol (basis 1,3-dioxolane charged) range up to 70 mol%. Total glycol products may comprise up to 77.1 wt% of the crude liquid.

Another surprising result of the process is that there is essentially no completing water gas shift or methanation activity.

The quantity of cobalt and silicon compounds to be used in the process of the invention may vary. The process is conducted in the presence of catalytically effective quantities of the catalyst components which gives the desired product in a reasonable yield. The reaction pro ceeds when employing as little as $10^{-2}$ weight %, and even lesser amounts of the cobalt compound, together with as little as $10^{-2}$ weight % of the silicon compound, based on the total weight of the reaction mixture. The upper concentration is dictated by a variety of factors including catalyst cost, partial pressures of carbon monoxide and hydrogen, operating temperature, etc. A cobalt compound concentration of from $10^{-2}$ to 30 weight % in conjunction with a silicon compound concentration of from $10^{-2}$ to 50 weight %, preferably $10^{-2}$ to 30 weight %, based on the total weight of the reaction mixture is generally desirable in the practice of this invention.

The solvent concentration can, for example, be from 10 to 95 weight %, based on total reaction mixture.

Particularly superior results are obtained when the above-noted components of the catalyst system are combined as follows on a molar basis: cobalt to silicon of 1:0.1 to 1:10 (Co:Si). With dioxolane as co-reactant the Co:Si mol ratio could be from 1:0.1 to 1:100. In contrast to the direct synthesis of ethylene glycol from synthesis gas (as for example in US-A-4 367 820) normally conducted at higher pressures than in this work, the use of a silicon compound in large excess over the cobalt compound, leads to a substantial suppression of glycol yield from syngas plus formaldehyde (see, for example, Table 1, Example 5).

The temperature which can be employed in the process of the invention may vary over a considerable range, depending upon experimental factors, including the choice of catalyst, pressure and other variables. A preferred range of operability is from 50 to 350°C. A narrower range of 100 to 220°C represents a particularly preferred temperature range.

The pressure employed may also vary over a considerable range, but in most cases is at least 3.5 MPa. The preferred operating range varies depending on co-reactant and solvent, and is form 7 to 28 MPa fro formaldehyde/cyclic ether, from 7 to 35 for formaldehyde and other solvents, and from 7 to 45 for 1,3-dioxolane, with or without one of the other solvents. Pressures above these maxima also provide useful yields of the desired product. The pressures referred to herein represent the total pressure generated by all the reactants, although they are substantially due to the carbon monoxide and hydrogen fractions. In the presence of formaldehyde or 1,3-dioxolane, the total pressures required for glycol syntheses using cobalt/silane-promoted catalyst systems are normally lower than those pressures required for direct glycol production from $CO/H_2$ (see, for example, US-A-4 367 820). This may in part be due to the fact that the formation of formaldehyde from synthesis gas (eq 3) is thermodynamically unfavourable under the range of operating conditions disclosed in these syntheses (see Stanford Research Institute Report 23A (Dec. 1978) entitled "Formaldehyde").

(3) $CO + H_2 \rightleftharpoons HCHO$

The relative amounts of carbon monoxide and hydrogen which can be initially present in the syngas mixture are variable, and these amounts may be varied over a wide range. In general, the mol ratio of $CO:H_2$ is in the range from 20:1 to 1:20, and preferably from 5:1 to 1:5, although ratios outside these ranges may also be employed with good results. Particularly in continuous operations, but also in batch experiments, the carbon monoxide-hydrogen gaseous mixture may also be used in conjunction with up to 50% by volume of one or more other gases. These other gases may include one or more inert gases such as nitrogen, argon and neon, or they may include gases that may, or may not, undergo reaction under carbon monoxide hydrogenation conditions, such as carbon dioxide, hydrocarbons, such as methane, ethane and propane, ethers such as dimethyl ether, methylethyl ether and diethyl ether or alkanols, such as methanol.

In all these syntheses, in order to achieve a high degree of selectivity the amounts of carbon monoxide, hydrogen and formaldehyde (or 1,3-dioxolane) present in the reaction mixture should be sufficient at least to satisfy the stoichiometry of the desired formation of ethylene glycol as shown in equations (1) and (2) above. Excess carbon monoxide and/or hydrogen over the stoichiometric amount may be present, if desired.

The most desired product of this synthesis, ethylene glycol (EG) will be formed in significant quantities (up to 9 wt% concentration in the crude liquid product) and up to 27 mol% selectivity (basis total formaldehyde charged) using the cobalt-silicon promoted catalyst system of this invention, and formaldehyde/cyclic ether. With formaldehyde and one of the other solvents, the EG yield can be up to 37 wt%, and selectivity up to 55 wt%. With dioxolane and another solvent, the EG yield can be up to 50 mol% (basis dioxolane) with selectivity to total glycol products (EG plus its ether) of 65 mol% or more. With dioxolane as both solvent and reactant, the EG yield can be up to 70 mol%, and selectivity to total glycol products exceeding 77 wt%. Also formed are significant amounts of diethylene glycol (DEG), propylene glycol (PG), together with derivatives such as the ethylene glycol monoalkyl ethers (e.g. ethylene glycol

monomethyl ether, EGMME). Selectivity to total glycol products (EG + DEG + PG + EGMME) may be from 50 to 77 wt%, depending upon the particular conditions, catalyst, solvent, etc. Lower monohydric alcohols (methanol and ethanol) are also present in the crude liquid product mix. Each of these products can be recovered from the reaction mixture by conventional means, e.g. fractional distillation in vacuo.

The novel process of the invention can be conducted in a batchwise, semi-continuous or continuous manner. The catalyst can be initially introduced into the reaction zone batchwise, or it may be continuously or intermittently introduced into such a zone during the course of the synthesis reaction. Operating conditions can be adjusted to optimize the formation of the desired ethylene glycol product, and said material may be recovered by methods known to the art, such as distillation, fractionation, extraction and the like. A fraction rich in the catalyst components may then be recycled to the reaction zone, if desired, and additional product generated.

The products have been identified in this work by one or more of the following analytical procedures: viz, gas-liquid phase chromatography (glc), gas chromatography/infrared spectroscopy (GC/IR), nuclear magnetic resonance (nmr) and elemental analyses, or a combination of these techniques. Analyses have, for the most part, been by parts by weight; all temperatures are in degrees centigrade and all pressures in megapascals (MPa).

When formaldehyde is employed (Examples 1 to 26) the yield of ethylene glycol (mole%) is estimated basis equation 1 using the formula:

$$\frac{\text{Total Ethylene Glycol Produced (mmole)}}{\text{Total Formaldehyde Charged (mmole)}} \times 100$$

Total glycol yield (mole%) are estimated basis:

$$\frac{\text{Total Glycol Products Generated [EG+EGMME+PG+DEG (mmole)]}}{\text{Total Formaldehyde charged (mmole)}} \times 100$$

Total product yield (wt%) is estimated basis:

$$\frac{\text{(Total Liquid + Solid Product, g)-Catalyst + Solvent- + Formaldehyde Charged, g)}}{\text{(Catalyst + Solvent + Formaldehyde- Charged, g)}} \times 100$$

When dioxolane is employed (Examples 27 to 45) the yield of ethylene glycol (mole%) is estimated, basis equation 2, using the formula:

$$\frac{\text{Total Ethylene Glycol Produced (mmole)}}{\text{[Total 1,3-dioxolane charged (mmole)] x2}} \times 100$$

Total liquid product increase (wt%) is estimated basis:

$$\frac{\text{(Total Liquid + Solid Product, g)-(Total Catalyst- + Solvent + 1,3-dioxolane Charged, g)}}{\text{(Total Catalyst + Solvent + 1,3-Dioxolane- Charged, g)}} \times 100$$

When 1,3-dioxolane is employed as both solvent and reactant, the yield of EG is estimated as:

$$\frac{\text{Total Ethylene Glycol Produced (mmole)}}{\text{/Total 1,3-dioxolane charged (mmole) x2}} \times 100$$

Total liquid product increase (wt%) is estimated basis:

$$\frac{\text{(Total Liquid + Solid Product, g)-(Total Catalyst-}}{\text{(Total Catalyst + 1,3-Dioxolane-}}$$

$$\frac{\text{+ 1,3-Dioxolane Charged, g)}}{\text{Charged, g)}} \times 100$$

To illustrate the process of the invention, the following examples are given. It is to be understood, however, that the examples are given in the way of illustration and are not to be regarded as limiting the invention in any way.

EXAMPLE 1

A 450 ml capacity reactor with glass liner was charged with a mixture of dicobalt octacarbonyl (12.0 mmol)Co, 2.052 g), triethylsilane (24.0 mmol Si, 2.790 g) and paraformaldehyde (0.1 mol, 3.0 g) in p-dioxane (15.0 g). The mixture was flushed with nitrogen, the reactor sealed, flushed with synthesis gas, pressurized to 18.7 MPa with $CO/H_2$ (1:2), and heated to 160°C with agitation. After four hours, the reactor was allowed to cool, the gas pressure (17.3 MPa) was noted, and the excess gas was sampled and vented. 25.3 g of brown liquid product was recovered, there was no solid precipitate at this stage.

Analysis (glc and Karl Fischer titration) of the liquid product shows it to contain:

8.1 wt% ethylene glycol (EG)
3.3 wt% ethylene glycol monomethyl ether (EGMME)
1.0 wt% propylene glycol(PG)
6.5 wt% diethylene glycol (DEG)
2.5 wt% methanol
0.8 wt% ethanol
66.5 wt% p-dioxane solvent
2.2 wt% water

Cobalt recovery in the liquid product was estimated to be 87% of that originally charged.

Analysis of the gas sample shows it to contain:

67% hydrogen
33% carbon monoxide
<0.1% carbon dioxide
<0.1% methane

Estimated yield of ethylene glycol is 1.66 g (26.8 mmol).

Estimated yield of ethylene glycol (basis formaldehyde charged) is 27 mol%.

Estimated yield of total glycol products (EG + PG + DEG + EGMME) is 51 mol%.

Total product yield is 11 wt%.

EXAMPLES 2 to 12

Examples 2 to 12 were conducted in the same way as Example 1. In every example dicobalt octacarbonyl was the cobalt-containing catalyst used and p-dioxane was employed as the solvent. Different halogen-free silicon-containing compounds were employed and the results in terms of weight percent of ethylene glycol, propylene glycol, diethylene glycol, glycol monomethyl ether, etc. in the crude liquid product are shown in Table 1.

It may be seen from an inspection of Table 1 that:

a) A variety of halogen-free silicon-containing compounds are effective promoters for the synthesis of ethylene glycol from synthesis gas plus formaldehyde, including triethylsilane, triphenylsilane, tricyclohexylsilane, diphenylsilane and hydroxytriphenylsilane.

b) For the dicobalt octacarbonyl-triethylsilane catalyst combination the highest ethylene glycol yields are realized at an initial Co:Si mole ratio of 1:2 (Example 1).

c) Ethylene glycol yields are substantially reduced, product yields are lower, and cobalt recovery is poorer, when a large excess of silane promoter ($Et_3SiH$) is added to solubilize the dicobalt octacarbonyl catalyst precursor (see Example 5), and the initial Si:Co molar ratio is >10.

TABLE 1

| Example | Composition | Liquid Product Composition (Wt%) EG | EGME | PG | DEG | MeOH | EtOH | $H_2O$ | Cobalt[b] Recov. (%) | Product[b] Yield (%) |
|---|---|---|---|---|---|---|---|---|---|---|
| 2 | $Co_2(CO)_8$-$2Et_3SiH$ | 6.2 | 2.3 | 0.9 | 5.5 | 1.6 | 1.0 | 7.6 | >99 | 15 |
| 3 | " -$Et_3SiH$ | 5.0 | 1.9 | 0.8 | 3.8 | 1.4 | 1.0 | 5.0 | >99 | 15 |
| 4 | " -$8Et_3SiH$ | 1.4 | 1.8 | 0.4 | 2.2 | 2.1 | 0.3 | 4.3 | 76 | 11 |
| 5 | " -$22Et_3SiH$ | 1.3 | 1.1 | 0.3 | 0.3 | 2.4 | | 0.1 | 45 | 7 |
| 6 | " -$4Ph_3SiH$ | (1.4<br>(6.1 | 4.7<br>5.8 | 0.5<br>0.7 | 3.1<br>4.7 | 2.0<br>3.6 | 0.8<br>1.0 | 3.0)<br>) | 92 | 8[c] |
| 7 | " -$4(C_6H_{11})_3SiH$ | (0.1<br>(7.1 | 0.9<br>4.1 | <br>0.4 | 1.8<br>8.1 | 1.1<br>3.0 | 0.3<br>0.9 | 5.7)<br>) | >99 | 8[c] |
| 8 | " -$4(EtO)_3SiH$ | 6.6 | 1.9 | 0.3 | 5.0 | 1.7 | 11.4 | 2.9 | d | 14[d] |
| 9 | " -$4Ph_2SiH_2$ | (1.7<br>(7.3 | 2.9<br>4.8 | 0.2<br>0.5 | 2.2<br>3.6 | 1.9<br>3.9 | 0.4<br>0.8 | 2.4)<br>5.7) | >99 | 11[c] |
| 10 | " -$4Ph(EtO)_3Si$ | 8.6[e] | 0.7 | 0.3 | 3.0 | 0.7 | 7.8 | 4.6 | >99 | 16 |
| 11 | " -$4Ph_3SiOH$ | ( 3.9<br>(11.3 | 5.3<br>4.6 | 0.2<br>0.7 | 3.8<br>6.7 | 1.0<br>1.7 | 0.7<br>1.1 | 2.3)<br>10.2) | | 13[c] |
| 12 | " -$Me_4$-Si | 4.8 | 1.0 | 0.7 | 1.8 | 0.5 | 0.5 | 7.9 | >99 | 23 |

a Reaction charge: Co, 12.0 mmol; Si, 12.0 mmol; $H_2CO$, 100 mmol; p-dioxane, 15.0 g. Run conditions: 160°C; 18.7 MPa; $CO/H_2$ (1:2) initial pressure; 4 hours.

b Cobalt recovery estimated basis cobalt recovered in solution at the end of run versus cobalt originally charged; total product yield calculated as described in text.

c Two-phase liquid product.

d Low liquid yield, considerable quantity of solid product.
e Glycol present as ethylene glycol monoethyl ether.

COMPARATIVE EXAMPLES 13 to 18

Examples 13 to 18 were conducted with the same reaction charge as Example 1 as well as with the same set of operating conditions. In these examples, however, halogen-containing silicon compounds were used. It is noted that the weight percentage of ethylene glycol in the product is much less.

## TABLE 2

| Example | Catalyst Composition[a] | Liquid Product Composition (wt%) EG | EGME | PG | DEG | MeOH | EtOH | $H_2O$ | Cobalt Recov. (%) | Product Yield[b] (wt%) |
|---|---|---|---|---|---|---|---|---|---|---|
| 13 | $Co_2(CO)_8$-4$Me_3$SiCl | 0.1 | 0.4 | | | 0.6 | | 1.5 | <5 | None[c] |
| 14 | " -4$Me_2$SiHCl | (0.6<br>(0.5 | | 0.2<br>0.3 | 0.3<br>0.4 | 1.4<br>2.7 | | 1.6)<br>8.3) | <5 | None[d,c] |
| 15 | " -4$Ph_3$SiCl | 0.3 | 2.6 | 0.2 | | 0.7 | | | <5 | None[c] |
| 16 | " -4$Me_2$Si$Cl_2$ | 0.2 | 0.1 | | | | | | <5 | None[c] |
| 17 | " -4$Et_3$SiCl | 0.1 | 0.1 | | | 0.8 | | 0.7 | <5 | None[c] |
| 18 | " -4$Me_3$SiI | | 0.1 | | | 0.1 | | 0.8 | | None |

a Reaction charge and operating conditions as per Table 1.

b Product yield calculated basis weight of catalyst-formaldehyde solution originally charged.

c Low liquid yield, considerable quantity of solid product.

d Two-phase liquid product.

COMPARATIVE EXAMPLE 19

Following the procedures of Example 1, the reactor was charged with a mixture of dicobalt octacarbonyl (12.0 mmol Co, 2.052 g), triethylsilane (129 mmol Si, 15.0 g) and paraformaldehyde (100 mmol, 3.0 g). After pressurizing to 18.7 MPa with $CO/H_2$ (1:2) and heating to 160°C with agitation for 4 hours, the reactor was allowed to cool, the gas pressure (17.5 MPa) was noted, and the excess gas sampled and vented. 23.2 g of brown liquid suspension was recovered.

Analysis of the liquid product fraction shows it to contain:

0.1 wt% ethylene glycol
0.5 wt% ethylene glycol monomethyl ether
1.1 wt% methanol
62.5 wt% p-dioxane solvent
0.9 wt% water

Analysis of the gas sample shows it to contain:

68% hydrogen
31% carbon monoxide

This example illustrates the poor yields of ethylene glycol obtained with the cobalt-silane catalyst system when the desired preparation from syngas and formaldehyde is conducted:

a) At initial Si:Co molar ratios of >10.

b) In the absence of added aliphatic ether solvent.

EXAMPLE 20

The procedure according to Example 1 was repeated, except that 1,2,4-trichlorobenzene (15.0 g) was used as solvent. After four hours, the reactor was allowed to cool, the gas pressure (11.3 MPa) was noted, and the excess gas was sampled and vented. 24.6 g of a two-phase liquid product was recovered, there was no solid precipitate at this stage.

Analysis (glc) of the less-dense liquid product (4 ml) shows it to contain:

24.5 wt% ethylene glycol (EG)
5.4 wt% ethylene glycol monomethyl ether (EGMME)
2.2 wt% propylene glycol (PG)
4.5 wt% diethylene glycol (DEG)
6.6 wt% methanol
0.4 wt% ethanol
31.7 wt% water
1.1 wt% 1,2,4-trichlorobenzene

Analysis by glc of the heavier liquid product (16 ml) shows it to contain:

90.8 wt% 1,2,4-trichlorobenzene
0.2 wt% methanol
0.6 wt% methyl formate
0.3 wt% ethylene glycol monomethyl ether

Analysis of the typical gas sample shows it to contain:

65% hydrogen
33% carbon monoxide
0.3 carbon dioxide

The ethylene glycol product and its derivatives may be recovered from the crude, aqueous-rich phase of the liquid product by fractional distillation in vacuo.

EXAMPLE 21

The procedure of Example 1 was followed, except that toluene (15.0 g) was used as solvent. After four hours, the reactor was allowed to cool, the gas pressure (13.2 MPa) was noted, and the excess gas was sampled and vented, 22.1 g of a two-phase liquid product was recovered, there was no solid precipitate at this stage.

Analysis (glc) of the more dense liquid product (3-4 ml) showed it to contain:

36.8 wt% ethylene glycol (EG)
7.1 wt% ethylene glycol monomethyl ether (EGMME)
2.4 wt% propylene glycol (PG)
8.2 wt% diethylene glycol (DEG)
18.3 wt% methanol
0.6 wt% ethanol
14.0 wt% water

Analysis of the lighter liquid product (25 ml) showed it to contain:

91% toluene
1.0% methanol
0.5% ethylene glycol
0.9% ethylene glycol monomethyl ether

Analysis of the typical gas sample showed it to contain:

67% hydrogen
32% carbon monoxide
0.1% methane

The ethylene glycol product and its derivatives may be recovered from the crude, aqueous rich phase of the liquid product by fractional distillation in vacuo.

EXAMPLES 22 to 26

The procedure of Example 21 was followed employing a variety of different solvents. The results are set out in Table 3 below.

TABLE 3

| Example | Catalyst Precursor[a] and Solvent | Liquid Product Composition (wt%) $H_2O$ | MeOH | EtOH | EGMME | EG | PG | DEG[d] | Solvent | Product Phase | Proportion Total Liquid Product (%) | Liquid Yield Increase (%) |
|---|---|---|---|---|---|---|---|---|---|---|---|---|
| 22 | $Co_2(CO)_8Et_3SiH$ m-xylene | 18.2 | 16.3 | 0.6 | 11.5 | 29.0 | 2.1 | 8.5 | | H | 11 | 9 |
| | | 0.9 | 1.1 | | 0.4 | 0.1 | | 0.1 | 90 | L | 89 | |
| 23 | $Co_2(CO)_8Et_3SiH$ tetrahydronaphthalene | 35.1 | 16.4 | 0.8 | 6.9 | 24.5 | 1.8 | 3.0 | | H | 12 | 5 |
| | | 0.6 | 0.3 | | 0.1 | 0.1 | 0.1 | | 94 | L | 88 | |
| 24 | $Co_2(CO)_8Et_3SiH$ p-xylene | 27.5 | 16.0 | 0.3 | 6.5 | 31.2 | 1.8 | 4.9 | 2.8 | H | 12 | 4 |
| | | 0.5 | 0.5 | | 0.2 | 0.1 | | | 93 | L | 88 | |
| 25 | $Co_2(CO)_8Et_3SiH$ 1,2,4-trichloro-benzene | 31.7 | 6.6 | 0.4 | 5.4 | 24.5 | 2.2 | 4.5 | 1.1 | L | 20 | 8 |
| | | | 0.2 | | 0.3 | | | | 91 | H | 80 | |
| 26 | $Co_2(CO)_8Ph_3SiH$ o-dichlorobenzene | 36.8 | 5.7 | 0.3 | 6.2 | 26.8 | 2.4 | 7.1[d] | d | L | 7 | 8 |
| | | 1.0 | 1.5 | 0.1 | 5.6 | 1.2 | 0.8 | | 82 | H | 93 | |

a Reactor Charge: Co, 12.0 mmol; Si, 24.0 mmol; $(HCO)_n$, 100 mmol; solvent, 15.0 g.
Run Conditions: 160°C; 18.7 MPa $CO/H_2$ (1:2) Initial Pressure, 4 hours.

b Product Phase: L, lower density phase; H, higher density phase.

c DEG fraction also contains alkyl silane.

d DEG, o-dichlorobenzene not resolved.

e PG, chlorobenzene not resolved.

EXAMPLE 27

A 450 ml capacity reactor with glass liner was charged with a mixture of dicobalt octacarbonyl (12.0 mmol Co, 2.052 g), triethylsilane (24.0 mmol Si, 2.790 g) and 1,3-dioxolane (200 mmol, 14.82 g), water (200 mmol, 3.60 g) and 1,2,4-trichlorobenzene (15.0 g). The mixture was flushed with nitrogen, the reactor sealed, flushed with synthesis gas, pressurized to 18.7 MPa with $CO/H_2$ (1:2), and heated to 160°C with agitation. After four hours, the reactor was allowed to cool, the gas pressure (15.95 MPa) was noted, and the excess gas was sampled and vented. 41.9 g of two-phase liquid product was recovered, there was no solid precipitate at this stage.

Analysis (glc) of the less dense liquid phase (20 ml) shows it to contain:
50.9 wt% ethylene glycol (EG)
3.4 wt% ethylene glycol monomethyl ether (EGMME)
0.7 wt% propylene glycol (PG)
3.2 wt% diethylene glycol (DEG)
1.8 wt% methanol
0.1 wt% ethanol
1.2 wt% 1,2,4-trichlorobenzene
20. 4 wt% water

Analysis (glc) of the heavier liquid phase (15 ml) shows it to contain:
91.4 wt% 1,2,4-trichlorobenzene
0.3 wt% ethylene glycol
0.2 wt% ethylene glycol monomethyl ether
8.1 wt% unidentified material

Analysis of the gas sample shows it to contain:
64% hydrogen
24% carbon monoxide
<0.1% carbon dioxide
<0.1% methane

Estimated yield of ethylene glycol is 174 mmol.

The estimated yield of ethylene glycol (basis 1,3-dioxolane charged) is 44 mol%.

The estimated liquid yield increase is 9.4 wt%.

EXAMPLES 28 to 39

Examples 28 to 39 were conducted in the same way as Example 27. In every example dicobalt octacarbonyl was the cobalt-containing catalyst used and 1,2,4-trichlorobenzene was employed as the solvent. The promoter used was triethylsilane, $Et_3SiH$, and the results in terms of weight percentage of ethylene glycol, propylene glycol, diethylene glycol, glycol monomethyl ether etc. in the crude liquid product are shown in Table 4.

It may be seen from an inspection of Table 4 that:

(a) Ethylene glycol is the predominant product fraction in many of these runs.

In Example 34, for example, the aqueous-glycol phase of the product fraction comprises:
59.2 wt% ethylene glycol, and
69.1 wt% total EG + PG + DEG + EGMME

Likewise, in Example 29, the aqueous-glycol phase comprised:
61.8 wt% ethylene glycol, and
65.5 wt% total EG + PG + DEG + EGMME

(b) Glycol synthesis has been demonstrated over a broad range of operating temperatures, pressures and 1,3-dioxolane/cobalt molar ratios.

TABLE 4

| Example | 1,3-dioxolane (mmol) | Water (mmol) | Temp (°C) | Pres. (MPa) | Liquid Product Composition (wt%) $H_2O$ | MeOH | EtOH | EGMME | EG | PG | DEG | Solvent | Dioxolane |
|---|---|---|---|---|---|---|---|---|---|---|---|---|---|
| 28 | 200 | 200 | 160 | 13.9 | (17.3<br>( 0.2 | 4.2<br>0.2 | 0.1 | 5.7<br>0.4 | 50.9<br>- | 0.3<br>0.1 | 1.7<br>1.6 | 5.3<br>91 | 0.6<br>0.6 |
| 29 | 200 | 200 | 160 | 7.0 | (14.9<br>( 0.1 | 4.0<br>0.2 | | 3.2<br>0.2 | 61.8<br>0.2 | 0.3 | 0.2<br>0.5 | 1.3<br>86 | 6.1<br>79 |
| 30 | 200 | 200 | 160 | 3.5 | (27.7<br>( 0.1 | 1.0<br>0.2 | 0.1 | 0.3 | 32.6<br>0.2 | | <br>0.3 | 11.7<br>53.7 | 26.0<br>38.6 |
| 31 | 200 | 200 | 140 | 13.9 | (23.2 | 1.6 | | 2.5<br>0.2 | 47.9 | 0.4 | 1.3<br>0.2 | 2.2<br>89.8 | 5.8<br>6.0 |
| 32 | 200 | 200 | 120 | 13.9 | (26.0 | 0.4 | | 0.8<br>0.1 | 30.0<br>0.1 | 0.6 | 0.6 | 2.4<br>70.5 | 20.9<br>19.4 |
| 33 | 200 | 200 | 100 | 13.9 | (32.3<br>( 0.6 | 0.1<br>0.1 | | 0.2<br>0.2 | 17.6 | 0.2<br>0.1 | <br>0.1 | 1.5<br>57.8 | 37.0<br>38.9 |
| 34 | 200 | 200 | 180 | 13.9 | (17.4<br>( 0.4 | 7.5<br>0.4 | 0.4 | 7.8<br>0.7 | 59.2<br>0.5 | 0.2 | 1.9<br>2.7 | 1.6<br>87.8 | -<br>0.1 |
| 35 | 200 | 200 | 200 | 13.9 | (19.6<br>( 0.2 | 7.7<br>1.0 | | 3.2<br>0.5 | 49.8<br>0.6 | 0.3 | 0.7<br>0.8 | 1.6<br>66.8 | 11.0<br>16.4 |
| 36 | 400 | 400 | 160 | 18.7[b] | (19.8 | 1.7<br>0.1 | | 2.1<br>0.2 | 55.4<br>0.2 | 0.7<br>0.2 | 2.0 | 3.2<br>89.4 | 0.2<br>0.2 |
| 37 | 200 | 200 | 180 | 34.6 | (21.0<br>( 0.2 | 3.3<br>0.2 | 0.9<br>0.1 | 5.6<br>0.6 | 48.8<br>0.3 | 0.4 | 4.6<br>1.2 | 1.1<br>86.6 | 0.3<br>0.1 |
| 38 | 200 | 200 | 180 | 34.6[c] | (21.6 | 1.1<br>0.2 | 2.4<br>0.5 | 5.0<br>0.8 | 48.2<br>0.1 | 0.5 | 6.5<br>2.8 | 5.7<br>83.7 | 2.1<br>0.8 |
| 39 | 200 | 200 | 180 | 55.3 | (20.0 | 1.7<br>0.1 | 1.0<br>0.1 | 4.6<br>0.1 | 49.9<br>0.3 | 0.8<br>0.3 | 7.0<br>1.0 | 2.0<br>89.1 | 0.7<br>0.3 |

a Charge: Co, 12.0 mmol; Si, 24.0 mmol;
Run Conditions $CO/H_2$, 1:2; 4 hours; constant pressure.
b Initial Pressure.
c Run for 18 hours.

Table 5 shows the volume of the liquid product in Examples 28 to 39. Also given is the ethylene glycol yield for each sample in mmols.

TABLE 5

| Example | Liquid Product Volume (ml) | Liquid Product Weight (g) | EG Yield (mmol) |
|---|---|---|---|
| 28 | 18)<br>15) | 39.6 | 156 |
| 29 | 16)<br>16) | 38.6 | 167 |
| 30 | 9)<br>21) | 36.1 | 47 |
| 31 | 19)<br>15) | 39.7 | 151 |
| 32 | 16)<br>17) | 38.3 | 78 |
| 33 | 11)<br>22) | 38.1 | 40 |
| 34 | 17)<br>16) | 38.8 | 166 |
| 35 | 14)<br>18) | 36.5 | 109 |
| 36 | 38)<br>12) | 62.0 | 392 |
| 37 | 19)<br>17) | 41.4 | 149 |
| 38 | 21)<br>18) | 42.1 | 154 |
| 39 | 20)<br>18) | 41.9 | 197 |

EXAMPLES 40 to 45

Examples 40 to 45 were conducted in the same manner as Example 27. The catalyst used in each case was dicobalt octacarbonyl and the promoter was triethylsilane. Different solvents were used and the effect upon ethylene glycol and total glycol production is shown in Table 6, while Table 7 illustrates the effect on liquid product volume/weight and gas composition.

It may be noted from an inspection of Tables 6 and 7 that:

(a) Both chlorinated aromatic solvents, such as o-dichlorobenzene, and hydrocarbyl ether solvents, such as p-dioxane and anisole, are found to be effective for the production of ethylene glycol in good selectivity and yields from syngas plus 1,3-dioxolane.

(b) There appears to be very little competing water-gas shift or methanation activity with this class of solvent-solubilized, cobalt-silane catalyst.

(c) Ethylene glycol/methanol ratios in the crude liquid product phase may exceed 25:1 in some cases (e.g. Example 43).

TABLE 6

| Example | Solvent | 1,3-dioxolane (mmol) | Water (mmol) | Temp (°C) | Pres. (MPa) | Liquid Product Composition (wt%) $H_2O$ | MeOH | EtOH | EGMME | EG | PG | DEG | Solvent | 1,3-dioxolane |
|---|---|---|---|---|---|---|---|---|---|---|---|---|---|---|
| 40 | p-dioxane | 100 | 100 | 160 | 18.7[b] | 8.9 | 0.9 | 0.6 | 3.4 | 13.3 | 0.2 | 8.4 | 61.3 | 0.2 |
| | | | | | | 0.5 | 0.2 | 0.1 | 0.3 | 0.2 | | 1.4 | 39.6 | 0.1 |
| 41 | " | 200 | 200 | 160 | 18.7[b] | 10.6 | 0.8 | | 0.6 | 30.7 | 0.2 | 4.1 | 48.0 | - |
| | | | | | | 0.4 | | | | 0.3 | 0.4 | 0.3 | 22/72 | - |
| 42 | Anisole | 100 | 100 | 160 | 18.7[b] | 16.8 | 1.7 | 0.3 | 8.4 | 32.1 | 2.9 | 18.4 | 10.3 | 0.1 |
| | | | | | | 3.2 | 0.2 | | 0.1 | 0.3 | 0.2 | 0.7 | 82.4 | |
| 43 | Anisole | 200 | 200 | 160 | 18.7[b] | 19.0 | 1.9 | 0.2 | 5.2 | 51.3 | 0.6 | 7.8 | 6.9 | - |
| | | | | | | 0.2 | 0.2 | | 0.7 | 0.9 | | 1.0 | 90 | - |
| 44 | Anisole | 200 | 200 | 160 | 13.9[d] | 15.7 | 3.6 | 0.2 | 7.3 | 50.0 | 0.2 | 6.2 | 7.9 | 0.2 |
| | | | | | | 0.3 | 0.4 | | 1.2 | 0.9 | 0.2 | 0.7 | 90 | 0.2 |
| 45 | o-dichloro-benzene | 100 | 100 | 160 | 18.7[b] | 14.5 | 2.6 | 0.3 | 7.5 | 45.0 | 0.6 | | 18.5 | 0.1 |
| | | | | | | 0.1 | 0.3 | | 0.9 | 0.7 | 0.2 | | 93 | - |

a Charge: CO, 12.0 mmol, Si, 24.0 mmol; Run Conditions $CO/H_2$ 1:2; 4 hours.

b Initial Pressure.

c Not determined.

d Constant pressure.

e No data.

TABLE 7

| Liquid Product | | Gas Composition (%) | | | |
|---|---|---|---|---|---|
| Volume (ml) | Weight (g) | $H_2O$ | CO | $CO_2$ | $CH_4$ |
| a a | 31.8 | 67 | 33 | 0.1 | 0.1 |
| 37) 3 | 43.0 | 65 | 35 | 0.1 | 0.1 |
| 11) 20) | 31.6 | 67 | 33 | 0.1 | |
| 20) 21) | 42.0 | 65 | 33 | | |
| 19) 20) | 40.7 | 63 | 36 | | 0.1 |
| 9) 17) | 30.2 | 63 | 33 | | |

EXAMPLE 46

A 450 ml capacity reactor with glass liner was charged with a mixture of dicobalt octacarbonyl (12.0 mmol Co, 2.052 g), triethylsilane (24.0 mmol, 2.790 g) in 1,3-dioxolane (400 mmol, 29.63 g) and water (200 mmol, 3.60 g). The mixture was flushed with nitrogen, the reactor sealed, flushed with synthesis gas ($CO/H_2$, 1:2), pressurized to 18.7 MPa with $CO/H_2$ (1:2), and heated to 160°C with agitation. After four hours, the reactor was allowed to cool, the gas pressure (12.3 MPa) was noted, and the excess gas was sampled and vented.

43.2 g of a red liquid, two phase, product was recovered. There was no solid precipitate at this stage.

Analysis of the liquid product shows the lighter phase (3.5 ml) to be primarily silane. The heavier phase (37 ml) was found to comprise:

69.1 wt% ethylene glycol (EG)
4.4 wt% ethylene glycol monomethyl ether (EGMME)
0.9 wt% propylene glycol (PG)
2.7 wt% diethylene glycol (DEG)
11.5 wt% water
1.1 wt% methanol
<0.1 wt% unreacted 1,3-dioxolane

Estimated conversion of the 1,3-dioxolane charged was>98%. Estimated amount of ethylene glycol produced was 449 mmol. Estimated amount of glycol products (EG + EGMME + PG + DEG) was 476 mmol.

Typical gas samples comprise:
65% hydrogen
35% carbon monoxide

Cobalt recovery in solution was >98%.

EXAMPLE 47

In this Example, the dicobalt octacarbonyl-triethylsilane catalyst is used at an initial cobalt:silicon molar ratio of Ca. 1:11.

The procedure of Example 46 was followed employing 15 g (129 mmol) of triethylsilane. After four hours, the reactor was allowed to cool, the gas pressure (11.1 MPa) was noted, and the excess gas was sampled and vented.

53.6 g of a two phase liquid product was recovered. There was no solid precipitate at this stage.

The lighter phase (19 ml, red liquid) was primarily silane. The heavier phase (34 ml) was found to comprise:

57.9 wt% ethylene glycol (EG)
8.8 wt% ethylene glycol monomethyl ether (EGMME)
0.7 wt% propylene glycol (PG)
5.3 wt% diethylene glycol (DEG)
10.2 wt% water
2.5 wt% methanol
0.3 wt% unreacted 1,3-dioxolane

EXAMPLE 48

In this example the differences include the mmols of water used, the operating pressure and the temperature. The pressure and temperature used constitute forcing conditions which allow for improved yields of glycol. It is noted that 1,3-dioxolane and water are used in a 1:1 molar mix.

The 450 ml capacity reactor was charged with a mixture of dicobalt octacarbonyl (12.0 mmol Co, 2.052 g), triethylsilane (24.0 mmol, 2.790 g) in 1,3-dioxolane (400 mmol, 29.63 g) and water (400 mmol, 7.2 g). The mixture was flushed with synthesis gas ($CO/H_2$, 1:2), sealed and pressurized to 13.9 MPa with $CO/H_2$ - (1:2), heated to 180°C with agitation and the pressure was raised to 34.6 MPa. Pressure was maintained at 34.6 MPa by intermittent addition of syngas (1:2) from a large surge tank. After four hours, the reactor was allowed to cool, and the excess gas was vented.

46.8 g of a two phase liquid product was recovered. There was no solid precipitate at this stage.

Analysis of the liquid product shows the lighter phase (2 ml red liquid) to be primarily silane, while the heavier phase (41 ml, red liquid) contains:

66.7 wt% ethylene glycol (EG)
2.9 wt% ethylene glycol monomethyl ether (EGMME)
0.9 wt% propylene glycol (PG)
2.6 wt% diethylene glycol (DEG)
19.9 wt% water
1.4 wt% methanol
0.2 wt% unreacted 1,3-dioxolane

Estimated conversion of the 1,3-dioxolane charged was 98%. Estimated amount of ethylene glycol produced is 481 mmol. Estimated amount of glycol products (EG + EGMME + PG + DEG) was 514 mmol.

Cobalt recovery in solution was 98%.

EXAMPLE 49

1,3-dioxolane (1151.4 g, 15.5 mol) was stirred under carbon monoxide purge for 30 minutes, dicobalt octacarbonyl (80.0 g, 0.234 mol) was added followed by addition of triethylsilane (108.9, 0.936 mol) from a dropping funnel. With a minimum of delay, deionized water (280.4 g, 15.5 mol) was added from a dropping funnel, with vigorous gas evolution being carefully controlled during the initial addition. After all the water had been added, the mixture was stirred under CO atmosphere for about one hour until gas evolution ceased, and filtered under a CO blanket.

Cobalt concentration in the resulting clear red solution = 2.96%. A 300 ml stirred-tank reactor, fitted with continuous gas and liquid feed pumps and the necessary temperature and pressure controls, was fed the liquid cobalt octacarbonyl-triethylsilane/aqueous 1,3-dioxolane solution prepared as above, at a rate of 16 ml/hr, from a ruska pump. Synthesis gas (2:1, $H_2/CO$) was also fed to the reactor at a rate of 42 stpl/hr. Reactor temperature was held at 180°C, and the total pressure was maintained at 27.7 MPa. The liquid product was collected in an ice-cooled receiver followed by two dry ice/acetone traps. The ruska pump was refilled from a larger feed tank containing the cobalt-silane-1,3-dioxolane solution under a CO atmosphere, as necessary.

Analysis of the liquid product effluent (22.7 g/hr by glc showed it to comprise:
67.8 wt% ethylene glycol (EG)
3.8 wt% ethylene glycol monomethyl ether (EGMME)
0.4 wt% propylene glycol (PG)
2.2 wt% diethylene glycol (DEG)
0.7 wt% methanol
13.8 wt% water
3.4 wt% unreacted 1,3-dioxolane.

## Claims

1. A process for the production of mixtures containing ethylene glycol by reacting carbon monoxide, hydrogen and formaldehyde or 1,3-dioxolane at a temperature of at least 50°C and a pressure of at least 3.5 MPa in the presence of a cobalt catalyst, characterised in that the catalyst is a homogeneous liquid catalyst comprising cobalt, a halogen-free silicon compound having at least one carbon-silicon bond, and a solvent comprising an aromatic hydrocarbon or halohydrocarbon, a halo aromatic ether, a cyclic ether, or an excess of 1,3-dioxolane reactant.

2. A process according to claim 1 characterised in that the silicon compound is triethyl silane, triphenyl silane, trimethyl silane, diphenyl silane, tricyclohexyl silane, tetramethyl silane, tetraethyl silane, hydroxy triphenyl silane, diethyl silane or tripropyl silane.

3. A process according to claim 1 or 2 characterised in that the formaldehyde is provided in the form of paraformaldehyde and the Co: Si: mol ratio is form 10:1 to 1:10.

4. A process according to any of claims 1 to 3 characterised in that the solvent is 1,4-dioxane.

5. A process according to any of claims 1 to 3 characterised in that the solvent is toluene, m-xylene, p-xylene or tetrahydronaphthalene.

6. A process according to any of claims 1 to 3 characterised in that the solvent is 1,2,4-trichlorobenzene, o-dichlorobenzene, bromobenzene or p-dibromobenzene.

7. A process according to any of claims 1 to 3 characterised in that the solvent is p-bromoanisole, m-chloroanisole or brominated diphenyl oxide.

8. A process according to claim 1 or 2 characterised in that 1,3-dioxolane is employed, and in that the Co: Si ratio is form 10:1 to 1:100.

9. A process according to claim 8 characterised in that the solvent is 1,2,4-trichlorobenzene, o-dichlorobenzene, bromobenzene, 1,3,5-tribromobenzene, p-bromotoluene, chlorobenzene or o-dibromobenzene.

10. A process according to claim 8 characterised in that 1,3-dioxolane is employed, and in that the solvent is p-dioxane or anisole.

11. A process according to claim 8 characterised in that 1,3-dioxolane is employed as solvent, and in that the liquid catalyst comprises $10^{-2}$ to 30 weight % of cobalt compound, $10^{-2}$ to 50 weight % of silicon compound, and up to 95 weight % of dioxolane.

European Patent Office

# EUROPEAN SEARCH REPORT

Application number

EP 85 30 8699

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,Y | US-A-4 362 820 (L. KAPLAN) * Column 1, lines 6-14; column 2, line 33 - column 3, line 34; column 4, line 37 - column 7, line 27 * --- | 1,2,5 | C 07 C 29/38<br>C 07 C 31/20 |
| Y | EP-A-0 078 616 (TEXACO) * Page 2, line 30 - page 3, line 14; page 4, line 23 - page 5, line 35; page 7, line 7 - page 8, line 5 * --- | 1,4 | |
| A | US-A-4 079 085 (R.G. WALL) * Columns 5,6; claims 1,2 * ----- | 1 | |
| | | | TECHNICAL FIELDS SEARCHED (Int Cl 4) |
| | | | C 07 C 29/00<br>C 07 C 31/00 |

The present search report has been drawn up for all claims

| Place of search | Date of completion of the search | Examiner |
|---|---|---|
| THE HAGUE | 18-06-1986 | KINZINGER J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document